**Europäisches Patentamt**

**European Patent Office**

**Office européen des brevets**

(19)

(11) Veröffentlichungsnummer: **0 064 546**
**B1**

(12) # EUROPÄISCHE PATENTSCHRIFT

(45) Veröffentlichungstag der Patentschrift:
**10.04.85**

(21) Anmeldenummer: **81903245.9**

(22) Anmeldetag: **13.11.81**

(86) Internationale Anmeldenummer:
**PCT/DE 81/00203**

(87) Internationale Veröffentlichungsnummer:
**WO 82/01645 (27.05.82** Gazette 82/14**)**

(51) Int. Cl.⁴: **A 61 B 17/58,** A 61 F 2/30

(54) **VORRICHTUNG ZUM ZUSAMMENHALTEN DER TEILE EINES MENSCHLICHEN SCHULTER-ECK-GELENKES.**

(30) Priorität: **14.11.80 DE 3043566**

(43) Veröffentlichungstag der Anmeldung:
**17.11.82 Patentblatt 82/46**

(45) Bekanntmachung des Hinweises auf die Patenterteilung:
**10.04.85 Patentblatt 85/15**

(84) Benannte Vertragsstaaten:
**AT CH DE FR GB LI LU NL SE**

(56) Entgegenhaltungen:
**BE - A - 692 263**
**CH - A - 578 864**
**GB - A - 1 006 766**

(73) Patentinhaber: **MECRON MEDIZINISCHE PRODUKTE GMBH, Nunsdorfer Ring 25-27, D-1000 Berlin 48 (DE)**

(72) Erfinder: **RAHMANZADEH, Rahim, Herthastrasse 11, D-1000 Berlin 33 (DE)**

(74) Vertreter: **Jander, Dieter, Dipl.-Ing. et al, Dipl.-Ing. Dieter Jander Dr.-Ing. Manfred Böning Patentanwäite Kurfürstendamm 66, D-1000 Berlin 15 (DE)**

## Beschreibung

Die Erfindung bezieht sich auf eine Vorrichtung zum Zusammenhalten der Teile eines menschlichen Schulter-Eck-Gelenkes.

Das Schulter-Eck-Gelenk ist das Gelenk zwischen dem Schlüsselbein und dem Acromium; das ist der obere Teil des Schulterblattes.

Aufgrund eines Unfalles kann das Schlüsselbein aus dem Acromium herausspringen.

Es ist eine Vorrichtung bekannt, die das Schlüsselbein am Acromium nach Wiederherstellung der Verbindung hält. Es handelt sich hierbei um eine etwa S-förmig gebogene Platte, die sowohl am Schlüsselbein als auch am Acromium befestigt wird und durch das Schulter-Eck-Gelenk hindurchläuft. Ihr Nachteil besteht darin, dass sie, weil sie einteilig ist, keine Bewegungen zwischen Schlüsselbein und Acromium zulässt und dass Beschädigungen im Schulter-Eck-Gelenk unvermeidbar sind.

Der Erfindung liegt die Aufgabe zugrunde, eine Vorrichtung der eingangs erwähnten Art zu schaffen, die Drehbewegungen des Schlüsselbeines relativ zum Acromium zulässt und Beschädigungen der Innenbereiche des Schulter-Eck-Gelenkes vermeidet.

Diese Aufgabe wird erfindungsgemäss gelöst durch eine erste Platte, die auf dem Schlüsselbein vorzugsweise oben befestigbar ist, eine zweite Platte, die auf dem Acromium vorzugsweise oben befestigbar ist, und ein Gelenk, über das beide Platten miteinander verbunden sind.

Eine solche Vorrichtung lässt Drehbewegungen des Schlüsselbeines relativ zum Acromium, insbesondere solche etwa um die Längsachse des Schlüsselbeines zu. (Im gesunden Fall kommen Drehbewegungen von 10-15° vor.) Andererseits laufen keine Teile der Vorrichtung durch das Schulter-Eck-Gelenk, so dass keine Beschädigungen desselben auftreten können.

Weitere Einzelheiten der Erfindung ergeben sich aus den Unteransprüchen und der Zeichnung. Darin zeigen:

Fig. 1 stilisiert von obenn gesehen, bezogen auf den stehenden Menschen, ein Schulter-Eck-Gelenk;

Fig. 2 eine Ausführungsform der erfindungsgemässen Vorrichtung;

Fig. 3 einen Schnitt nach der Linie III-III der Fig. 2;

Fig. 4 eine Seitenansicht der zweiten Platte der erfindungsgemässen Vorrichtung;

Fig. 5 das Schulter-Eck-Gelenk mit angesetzter erfindungsgemässer Vorrichtung und

Fig. 6 eine andere Ausführungsform der zweiten Platte.

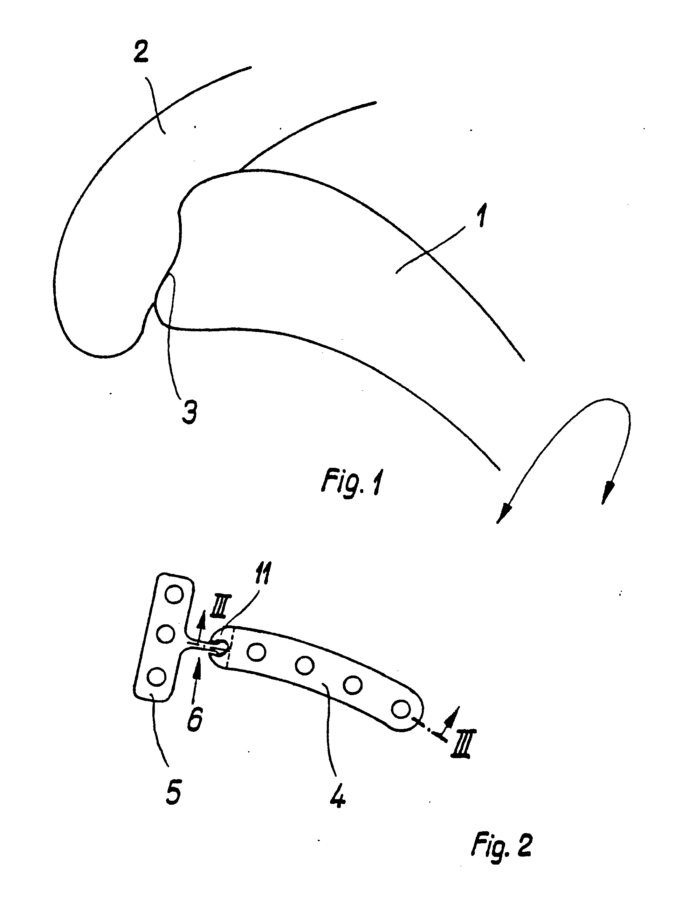

In Fig. 1 ist mit 1 das Schlüsselbein, mit 2 das Acromium und mit 3 das Schulter-Eck-Gelenk bezeichnet. Das Schlüsselbein kann Drehbewegungen um etwa seine Längsachse von 10-15° vollführenn (s. eingezeichneten Pfeil). Aufgrund eines Unfalles kann das Schlüsselbein 1 aus seiner Sollage herausspringen und ragt dann, bezogen auf den stehenden Menschen, schräg nach oben. Die erfindungsgemässe Vorrichtung gestattet es, das in seine Sollage zurückgedrückte Schlüsselbein zu halten.

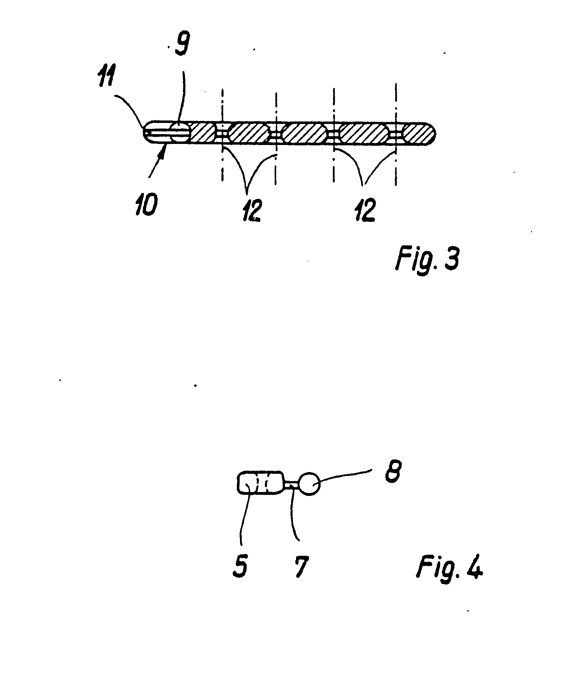

Die in den Fig. 2 bis 4 veranschaulichte Vorrichtung besteht aus einer ersten Platte 4, die am Schlüsselbein 1 oben befestigt wird, einer zweiten Platte 5, die am Acromium 2 oben befestigt wird und einem Gelenk 6 zwischen der Platte 5 und der Platte 4. Letzteres besteht aus einem Stiel 7, der an der Platte 5 befestigt ist, und einer Kugel 8, die am Ende des Stieles 7 sitzt. Die Kugel 8 ruht in einer kugelförmigen Ausnehmung 9 der Platte 4, die den inneren Teil eines Einschnittes 10 bildet. Das linke Ende der Platte 4 ist mit einem Schlitz 11 versehen, der das Einsetzen der Kugel 8 in die Ausnehmung 9 erleichtert. Diese wird von links her durch den Einschnitt 10 in die Ausnehmung 9 eingeschoben. Die Bohrungen 12 in der Platte 4, durch welche Knochenschrauben laufen, sind beidseitig konisch ausgeformt, so dass die Platte 4 sowohl auf der rechten Schulter wie auf der linken Schulter angesetzt werden kann.

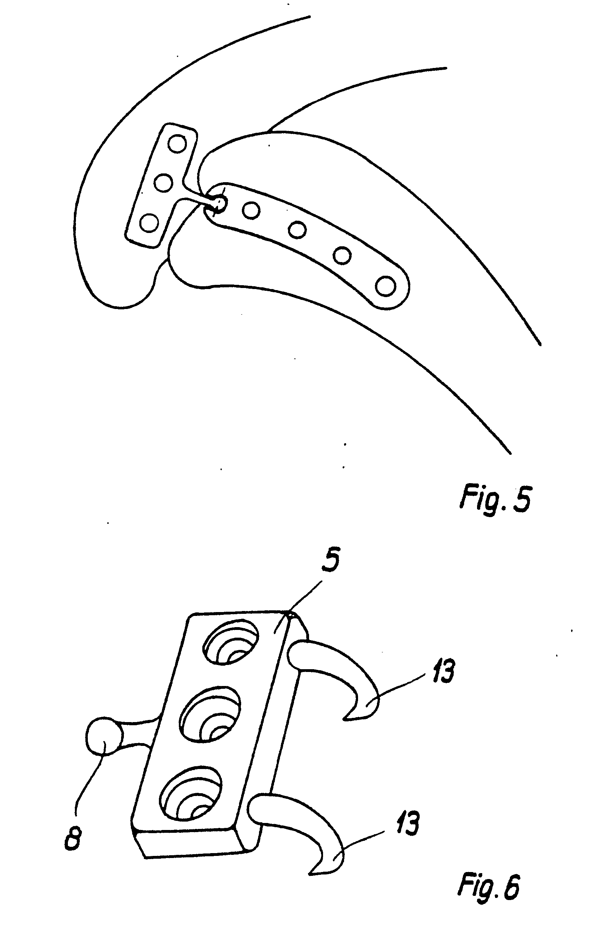

Fig. 5 veranschaulicht, wie die erfindungsgemässe Vorrichtung auf dem Acromium und dem Schlüsselbein sitzt.

Fig. 6 zeigt eine Abwandlung der Platte 5. Auf der der Kugel 8 abgewandten Seite sind zwei Haken 13 vorgesehen, die in den Knochen 2 (s. Fig. 1) eindringen können. Diese Haken sind in mehrfacher Hinsicht vorteilhaft: Sie geben der Platte 5 schon dann einen Halt, wenn die Schrauben noch nicht in den Knochen eingezogen worden sind. Ferner halten sie die Platte 5 zusätzlich zu den Schrauben, was insofern bedeutsam ist, als der Knochen dünnwandig ist.

## Patentansprüche

1. Vorrichtung zum Zusammenhalten der Teile eines menschlichen Schulter-Eck-Gelenkes, gekennzeichnet durch eine erste Platte (4), die auf dem Schlüsselbein (1) vorzugsweise oben befestigbar ist, eine zweite Platte (5), die auf dem Acromium (2) vorzugsweise oben befestigbar ist, und ein Gelenk (6), über das beide Platten (4, 5) miteinander verbunden sind.

2. Vorrichtung nach Anspruch 1, dadurch gekennzeichnet, dass die Platten (4, 5) von länglicher Form sind und dass ihre Längsachsen etwa senkrecht zueinander stehen.

3. Vorrichtung nach Anspruch 2, dadurch gekennzeichnet, dass die zweite Platte (5) seitlich in der Mitte einen vorzugsweise um 90° abragenden Stiel (7) mit einer Kugel (8) an dessen Ende aufweist und dass die erste Platte (4) im Bereich einer kurzen Seite eine Kugelaufnahme (9) aufweist, in der die Kugel (8) ruht.

4. Vorrichtung nach Anspruch 3, dadurch gekennzeichnet, dass von der Stirnseite der ersten Platte (4) her ein Einschnitt (10) in die Platte (4) senkrecht zu den grössten Flächen der Platte ragt, dessen Grund (9) teilkugelförmig ausgeformt ist, wobei sich die Kugel (8) in dem Grund (9) des Einschnittes (10) und der Stiel (7) im übrigen Teil des Einschnittes (10) befindet.

5. Vorrichtung nach Anspruch 4, dadurch gekennzeichnet, dass das Ende der ersten Platte (4) mit einem bis zum Grund des Einschnittes (10) reichenden Schlitz (11) versehen ist, der senkrecht zum Einschnitt (10) verläuft.

6. Vorrichtung nach einem oder mehreren der Ansprüche 1 bis 5, dadurch gekennzeichnet, dass diese so ausgebildet ist, dass sie sowohl auf der rechten wie auf der linken Schulterseite ansetzbar ist.

7. Vorrichtung nach Anspruch 6, dadurch gekennzeichnet, dass Löcher (12) in der ersten Platte (4) zur Aufnahme der Köpfe von Knochenschrauben beidseitig konisch ausgeformt sind.

8. Vorrichtung nach einem oder mehreren der Ansprüche 1 bis 7, dadurch gekennzeichnet, dass die erste Platte (4) in der Befestigungsebene leicht gekrümmt ist.

9. Vorrichtung nach einem oder mehreren der Ansprüche 1 bis 8, dadurch gekennzeichnet, dass die zweite Platte (5) rechteckig ausgebildet ist.

10. Vorrichtung nach einem oder mehreren der Ansprüche 1 bis 9, dadurch gekennzeichnet, dass die zweite Platte (5) fest mit ihr verbundene Organe (13) aufweist, über die sie mit dem Knochen (2) verbunden werden kann.

11. Vorrichtung nach Anspruch 10, dadurch gekennzeichnet, dass die Organe zwei Haken (13) sind, die an der der ersten Platte (4) abgewandten Seite der zweiten Platte (5) befestigt sind und in den Knochen (2) eindringen können.

## Claims

1. Device for holding the parts of the joint of a human shoulder together, characterised by a first plate (4) which can be fixed to the collar bone (1), preferably to the top, a second plate (5) which can be fixed to the acromium (2), preferably to the top, and a joint (6) by means of which the two plates (4, 5) are mutually connected.

2. Device according to Claim 1, characterised in that the plates (4, 5) are of elongate shape and that their longitudinal axes are approximately perpendicular to one another.

3. Device according to Claim 2, characterised in that the second plate (5) has, laterally in the middle, a stem (7) projecting preferably at an angle of 90° and having a spherical head (8) at its end, and that the first plate (4) has, in the region of a short side, a head fitting (9) in which the spherical head (8) rests.

4. Device according to Claim 3, characterised in that an incision (10) extends from the end face of the first plate (4) into the plate (4) perpendicular to the largest areas of the plate, the base (9) of the incision having the shape of part of a sphere, and the spherical head (8) being located in the base (9) of the incision (10) and the stem (7) being located in the remaining part of the incision (10).

5. Device according to Claim 4, characterised in that the end of the first plate (4) is provided with a slot (11) which extends down to the base of the incision (10) and runs perpendicular to the incision (10).

6. Device according to one or more of Claims 1 to 5, characterised in that it is constructed in such a way that it can be attached either on the right-hand side or the left-hand side of the shoulder.

7. Device according to Claim 6, characterised in that holes (12) are formed conically on both sides in the first plate (4), for receiving the heads of bone screws.

8. Device according to one or more of Claims 1 to 7, characterised in that the first plate (4) is slightly curved in the plane of fixing.

9. Device according to one or more of Claims 1 to 8, characterised in that the second plate (5) is of rectangular shape.

10. Device according to one or more of Claims 1 to 9, characterised in that the second plate (5) has elements (13) which are firmly connected thereto and by means of which it can be connected to the bone (2).

11. Device according to Claim 10, characterised in that the elements are two hooks (13) which are fixed to that side of the second plate (5) which faces away from the first plate (4), and can penetrate into the bone (2).

## Revendications

1. Dispositif pour maintenir assemblés les éléments d'une articulation d'épaule humaine, caractérisé par une première plaque (4) qui peut être fixée sur la clavicule (1), de préférence au dessus, une deuxième plaque (5) qui peut être fixée sur l'acromion (2) de préférence au dessus, et une articulation (6) par l'intermédiaire de laquelle les deux plaques (4, 5) peuvent être reliées l'une à l'autre.

2. Dispositif selon la revendication 1, caractérisé en ce que les plaques (4, 5) sont de forme allongée et en ce que leurs axes longitudinaux sont à peu près perpendiculaires entre eux.

3. Dispositif selon la revendication 2, caractérisé en ce que la deuxième plaque (5) présente latéralement, en son milieu, une tige (7) qui fait saillie de préférence à 90°, munie d'une boule (8) à son extrémité et en ce que la première plaque (4) présente dans la région d'un bord étroit un logement de rotule (9) dans lequel repose la rotule (8).

4. Dispositif selon la revendication 3, caractérisé en ce que en partant de la face frontale de la première plaque (4), une entaille (10) pénètre dans la plaque (4) perpendiculairement aux grandes surfaces de cette plaque, le fond (9) de cette entaille étant de forme partiellement sphérique, la rotule (8) se trouvant dans le fond (9) de l'entaille (10) et la tige (7) dans la partie restante de l'entaille (10).

5. Dispositif selon la revendication 4, caractérisé en ce que l'extrémité de la première plaque (4) est munie d'une fente (11) qui se prolonge jusqu'au fond de l'entaille (10) et qui s'étend perpendiculairement à l'entaille (10).

6. Dispositif selon l'une ou plusieurs des revendications 1 à 5, caractérisé en ce qu'il est de configuration telle qu'il puisse être monté aussi bien sur le côté de l'épaule droite que sur le côté de l'épaule gauche.

7. Dispositif selon la revendication 6, caractérisé en ce que des trous (12) sont formés avec une forme conique des deux côtés dans la première plaque (4) pour recevoir les têtes des vis à os.

8. Dispositif selon l'une ou plusieurs des revendications 1 à 7, caractérisé en ce que la première plaque (4) est légèrement incurvée dans le plan de la fixation.

9. Dispositif selon l'une ou plusieurs des revendications 1 à 8, caractérisé en ce que la deuxième plaque (5) est de configuration rectangulaire.

10. Dispositif selon l'une ou plusieurs des revendications 1 à 9, caractérisé en ce que la deuxième plaque (5) présente des organes (13) qui lui sont fixés rigidement et par l'intermédiaire desquels elle peut être fixée à l'os (2).

11. Dispositif selon la revendication 10, caractérisé en ce que les organes sont deux crochets (13) qui sont fixés à la face de la deuxième plaque (5) qui est à l'opposé de la première plaque (4) et qui peuvent pénétrer dans l'os (2).

Fig. 1

Fig. 2

**Fig. 3**

**Fig. 4**

*Fig. 5*

*Fig. 6*